# EUROPEAN PATENT SPECIFICATION

(11) **EP 4 365 963 B1**
(45) Date of publication and mention of the grant of the patent: **18.11.2026**
(21) Application number: 22832778.9
(22) Date of filing: 10.06.2022
(51) Int. Cl.: H10H 20/825, H10H 20/84, H10W 90/00, G01J 1/04, G01J 1/42, A61L 2/10, A61N 5/06, G01J 1/02, G02B 5/28, H10F 77/40

(54) **ULTRAVIOLET RAY RECEIVING AND EMITTING DEVICE**
ULTRAVIOLETTSTRAHLENEMPFANGS- UND -SENDEVORRICHTUNG
DISPOSITIF DE RÉCEPTION ET D'ÉMISSION DE RAYONS ULTRAVIOLETS

(30) Priority: 29.06.2021 JP 2021107650; 22.11.2021 JP 2021189554
(43) Date of publication of application: 08.05.2024
(73) Proprietor: Asahi Kasei Kabushiki Kaisha, Tokyo 1000006 (JP)
(72) Inventor: SATO Kosuke, Tokyo 100-0006 (JP)
(74) Representative: Hoffmann Eitle
(86) International application number: PCT/JP2022/023509
(87) International publication number: WO 2023/276621

(56) References cited:
- EP-A1- 4 317 361
- WO-A1-2022/203882
- CN-A- 110 828 644
- JP-A- 2002 350 228
- JP-A- 2003 188 407
- JP-A- 2019 187 727
- JP-A- 2020 068 283
- US-A- 2 349 754
- US-A1- 2018 373 157
- LV ZUNXIAN ET AL: "A High Responsivity and Photosensitivity Self-Powered UV Photodetector Constructed by the CuZnS/Ga 2 O 3 Heterojunction", vol. 10, no. 5, 4 December 2022 (2022-12-04), DE, XP093208239, ISSN: 2196-7350, Retrieved from the Internet <URL:https://onlinelibrary.wiley.com/doi/full-xml/10.1002/admi.202202130> DOI: 10.1002/admi.202202130

## Description

### Technical Field

The present disclosure relates to an ultraviolet ray receiving and emitting device.

### Background Art

In the related art, an ultraviolet sterilization device that emits ultraviolet light in a wavelength range of a wavelength of 200 to 230 nm to perform a sterilization treatment or treatment of the skin (for example, PTL 1). Because it has been found that ultraviolet light in a wavelength range of a wavelength of 230 to 300 nm has an adverse effect on a human body, the ultraviolet sterilization device includes a bandpass filter that blocks ultraviolet light in the wavelength range of 230 to 300 nm and further includes an ultraviolet absorption member that absorbs the ultraviolet light in a wavelength range of 230 to 300 nm in the ultraviolet sterilization device. In this way, the ultraviolet sterilization device is devised to suppress emission of the ultraviolet light in a wavelength range of a wavelength of 230 to 300 nm to the outside.

Further, because ultraviolet ray having a wavelength of 220 nm or less generates ozone gas that is harmful to a human body, a method for removing deposits on a roll surface by using a low-pressure UV lamp which eliminates ultraviolet ray having a wavelength of 220 nm or less is disclosed in the related art (For example, PTL 2). From these findings, it is clear that ultraviolet light that is harmful to a human body includes ultraviolet light having a wavelength range of 220 nm or less and ultraviolet light having a wavelength range of a wavelength of 230 to 300 nm.

### Citation List

### Patent Literatures

PTL 1: WO 2018/131582A1
PTL 2: WO 2018/012052A1

EP 4 317 361 A1 discloses an inspection tool capable of inspecting whether or not a light which inactivates a virus has been radiated and whether or not a light which is harmful to the human body has been radiated.

US 2 349 754 A discloses a method and instrument for measuring ultra-violet radiations and particularly for measuring the output of lamps which generate bactericidal radiations.

US 2018/3731 57 A1 discloses a method of operating an UV light source and also includes sensing the UV light emitted by the UV light source to measure an optical parameter of the UV light such as antimicrobial efficacy.

LV ZUNXIAN ET AL: "A High Responsivity and Photosensitivity Self- Powered UV Photodetector Constructed by the CuZnS/Ga 2 0 3 Heterojunction", ADVANCED MATERIALS INTERFACES, vol. 10, no. 5, 4 December 2022 (2022-12-04) discloses a heterojunction photodetector and a method of building thereof.

### Summary of Invention

### Technical Problem

However, even when an ultraviolet ray emitting device and a method of eliminating ultraviolet ray from emitted light described above are used, it is impossible to completely remove ultraviolet light in a wavelength range of a wavelength of 230 nm or more. For example, when the above-described ultraviolet ray emitting device is used, ultraviolet light in a wavelength range of of a wavelength of 230 nm or more may be emitted to the outside due to degradation of a bandpass filter or an ultraviolet absorption member. Since ultraviolet light having a wavelength of 230 nm to 400 nm is not visible, even when a human body is exposed to harmful ultraviolet ray, it cannot be noticed.

In order to solve the above-described problem, the present disclosure aims to provide an ultraviolet ray receiving and emitting device that efficiently detects ultraviolet light in a wavelength band of a wavelength of 230 nm to 400 nm to detect a risk, in a space where light in a wavelength band having a wavelength of a wavelength of 220 nm or more and less than 230 nm is used when ultraviolet light that is harmful to a human body in a wavelength band of a wavelength of 230 nm to 400 nm is emitted.

### Solution to Problem

This object is accomplished by the subject-matter of claim 1. The dependent claims concern particular embodiments.

### Advantageous Effects of Invention

According to the present disclosure, there is provided an ultraviolet ray receiving and emitting device that efficiently detects ultraviolet light in a wavelength band of a wavelength of 230 nm to 400 nm to detect a risk, in a space where light in a wavelength band having a wavelength band of a wavelength of 220 nm or more and less than 230 nm is used when ultraviolet light that is harmful to a human body in a wavelength band of a wavelength of 230 nm to 400 nm is emitted.

### Brief Description of Drawings

FIG. 1 is a cross-sectional view schematically illustrating a configuration example of an ultraviolet ray receiving and emitting device according to an embodiment of the present disclosure;
FIG. 2 is a cross-sectional view illustrating a configuration example of an ultraviolet ray receiving device that configures an ultraviolet ray receiving and emitting device, according to an embodiment of the present disclosure;
FIG. 3 is a cross-sectional view illustrating another configuration example of an ultraviolet ray receiving device that configures an ultraviolet ray receiving and emitting device, according to an embodiment of the present disclosure; and
FIG. 4 is a cross-sectional view schematically illustrating a configuration example of an ultraviolet ray emitting device that configures an ultraviolet ray receiving and emitting device, according to an embodiment of the present disclosure.

### Description of Embodiments

Hereinafter, an ultraviolet ray receiving and emitting device according to the present disclosure is described. Further, not all combinations of characteristics described are essential for solution of the invention.

### 1. Ultraviolet ray receiving and emitting device

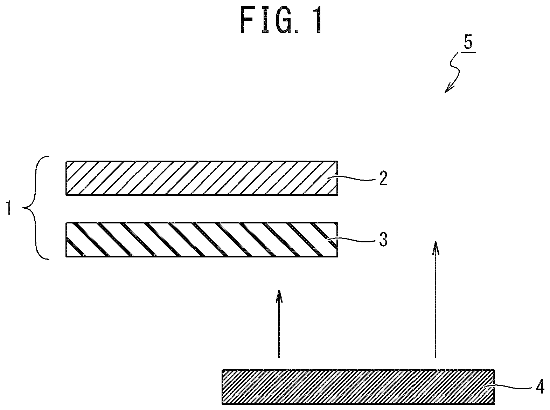

Hereinafter, an ultraviolet ray receiving and emitting device 5 according to an embodiment of the present disclosure is described with reference to FIGS. 1 to 4.

The ultraviolet ray receiving and emitting device 5 includes an ultraviolet ray receiving device 1 including a semiconductor element capable of receiving ultraviolet light, and a light source 4 which is a semiconductor element capable of emitting ultraviolet light.

### (1.1) Configuration of ultraviolet ray receiving and emitting device

The ultraviolet ray receiving and emitting device 5 of the present embodiment includes the light source 4 that emits light having a wavelength of 220 nm or more and less than 230 nm, and the ultraviolet ray receiving device 1 including a light receiving portion 2 and a filter 3. That is, the ultraviolet ray receiving and emitting device 5 illustrated in FIG. 1 is configured by the light source 4 and the ultraviolet ray receiving device 1 including the light receiving portion 2 and the filter 3.

The light receiving portion 2 is a sensor having sensitivity to both of light in both a wavelength band of 220 nm or more and less than 230 nm and light in a wavelength band of 230 nm or more and less than 400 nm. The filter 3 has a function of attenuating light in a wavelength band of 220 nm or more and less than 230 nm and transmitting light in a wavelength band of 230 nm or more and less than 320 nm therethrough. The ultraviolet light is incident on the light receiving portion 2 through the filter 3.

Hereinafter, respective structures of the ultraviolet ray receiving device 1 and the light source 4 that configure the ultraviolet ray receiving and emitting device 5 are described in detail.

### (1.2) Ultraviolet ray receiving device

### <Light Receiving Portion>

A type and a shape of a sensor are not limited in particular as long as the light receiving portion 2 is a sensor having sensitivity to light in both a wavelength band of 220 nm or more and less than 230 nm and a wavelength band of 230 nm or more and less than 400 nm. Specifically, the light receiving portion 2 can include a photomultiplier tube, a silicon photodiode, and an optical sensor formed of a material such as aluminum gallium nitride (AlGaN). The light receiving portion 2 is preferably an optical sensor formed of a material such as AlGaN, which has high light receiving sensitivity. Particularly, an optical sensor of a metal-semiconductor-metal (MSM) type having a metal-semiconductor-metal current path has extremely high light receiving sensitivity in a wavelength of 230 nm or more and less than 400 nm and is not affected by visible light, and thus, the optical sensor is preferable as an optical sensor of the present disclosure.

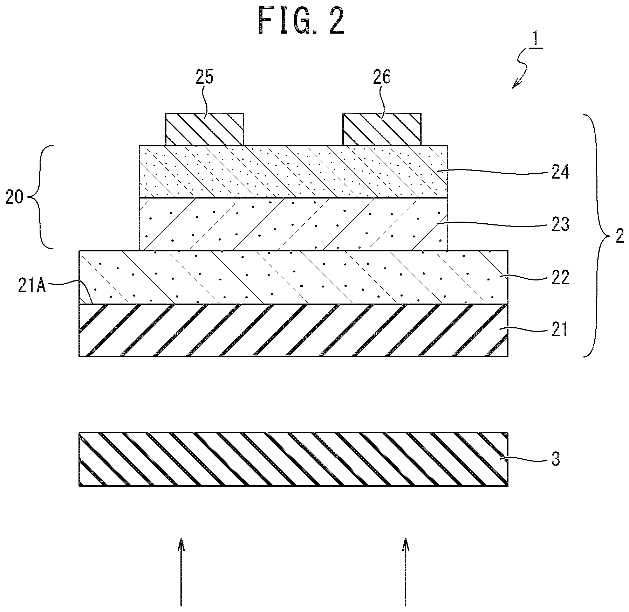

FIG. 2 illustrates an example of the ultraviolet ray receiving device 1 which is an optical sensor of an MSM type.

The light receiving portion 2 includes a substrate 21, semiconductor stacked portions 22, 23, and 24, and electrodes 25 and 26, and has sensitivity to light in both a wavelength band of 220 nm or more and less than 230 nm and a wavelength band of 230 nm or more and less than 400 nm. A shape of the substrate 21 is not limited in particular as long as the semiconductor stacked portion 22 can be disposed on a first main surface 21A.

The optical sensor of an MSM type may have a structure in which a current flows inside a solid of conductive AlGaN and may have a structure in which a current flows by using a two-dimensional electron gas or a two-dimensional hole gas at a stacked interface of AlGaN.

The light receiving portion 2 may be configured with a single optical sensor element, may be connected to a plurality of elements, or the plurality of elements may be discretely disposed therein.

When an optical sensor formed of a material such as AlGaN is used as the light receiving portion 2, the optical sensor is not limited to an optical sensor of an MSM type. Specifically, the optical sensor may be a photodiode of a PN-type in which a p-type semiconductor and an n-type semiconductor are stacked, a PIN type, or an avalanche type. When a photodiode of a PIN type is used, a layer that reacts with light may be a single layer or a multiple quantum well layer. Furthermore, a gate electrode may be used, or p-AlGaN or p-GaN may be disposed on an uppermost layer of a semiconductor stacked portion as a gate of an optical sensor. When a gate electrode uses a gate semiconductor, the gate electrode may have a MIS-type structure that is bonded to a semiconductor through an insulator.

Further, the light receiving portion 2 may be an optical sensor that uses a material such as diamond, gallium oxide, or MgZnO.

The light receiving portion 2 may have sensitivity to only a partial wavelength in a wavelength band of 220 nm or more and less than 230 nm or the entire wavelength band. Here, "having sensitivity" represents that, when light is incident on the light receiving portion 2, there is a change by comparing a current or a voltage converted from light by the light receiving portion 2 with a current or a voltage output from light receiving portion 2 when light is not incident. The light receiving portion 2 may have sensitivity to only a part of a wavelength band of 230 nm or more and less than 400 nm or may have sensitivity to the entire wavelength band. From the viewpoint of detecting light that is harmful to a human body without leakage, it is preferable to have sensitivity in the entire wavelength band.

The light receiving portion 2 may have sensitivity to light having a wavelength of 200 nm or more and less than 220 **nm.** By having sensitivity to the light having the wavelength of 200 nm or more and less than 220 nm, it is possible to detect whether ultraviolet ray that generates ozone is emitted.

Harmfulness of ultraviolet ray to a human body increases in the order of a wavelength band of 230 nm or more and less than 240 nm, a wavelength band of 240 nm or more and less than 250 nm, and a wavelength band of 250 nm or more and less than 280 nm, and harmfulness of the ultraviolet ray to the human body also increases in the order of a wavelength band of 320 nm or more and less than 400 nm and a wavelength band of 280 nm or more and less than 320 nm. Therefore, the light receiving portion 2 may have different sensitivities in a wavelength band having a wavelength of 230 nm or more and less than 240 nm, a wavelength of 240 nm or more and less than 250 nm, a wavelength of 250 nm or more and less than 280 nm, a wavelength of 280 nm or more and less than 320 nm, and a wavelength of 320 nm or more and less than 400 nm. Here, "sensitivities are different" means that amounts and ratios of changes are different from each other by comparing a current or a voltage converted from light by the light receiving portion 2 when light is incident on the light receiving portion 2 with a current or a voltage output from the light receiving portion 2 when light is not incident thereon.

The light receiving portion 2 preferably has the highest sensitivity to ultraviolet ray in a wavelength band of a wavelength of 250 nm or more and less than 280 nm, which is the most harmful to a human body. From this point of view, the light receiving portion 2 preferably has sensitivity to light having a wavelength of 230 nm or more and less than 240 nm, more preferably has sensitivity to light having a wavelength of 230 nm or more and less than 250 nm, more preferably has sensitivity to light having a wavelength of 230 nm or more and less than 280 nm, and still more preferably has sensitivity to light having a wavelength of 230 nm or more and less than 320 nm.

Further, light having a wavelength of 320 nm or more and less than 400 nm is less harmful to a human body than ultraviolet light in a wavelength band of a wavelength of 230 nm or more and less than 320 nm. Therefore, spectral sensitivity (A/W/nm) of ultraviolet light in a wavelength band of a wavelength of 320 nm or more and less than 400 nm may be lower than spectral sensitivity (A/W/nm) of ultraviolet light in a wavelength band of a wavelength of 230 nm or more and less than 320 nm.

Further, a plurality of elements having different sensitivities in a wavelength band of a wavelength of 230 nm or more and less than 240 nm, a wavelength of 240 nm or more and less than 250 nm, a wavelength of 250 nm or more and less than 280 nm, a wavelength of 280 nm or more and less than 320 nm, and a wavelength of 320 nm or more and less than 400 nm may be used. This may be achieved, for example, by using a plurality of elements of a sensor of an MSM type formed by using AlGaN as a material and changing an Al composition ratio of AlGaN.

From the viewpoint of making it possible to detect ultraviolet light that is harmful to a human body even with weak light, spectral sensitivity of ultraviolet light having a wavelength of 230 nm or more and less than 400 nm is preferably greater than 0.1 A/W/nm. From the viewpoint of making it possible to detect ultraviolet light that is harmful in particular to the human body even with weak light, spectral sensitivity of ultraviolet light having a wavelength of 250 nm or more and less than 280 nm is preferably greater than 0.1 A/W/nm. This is to efficiently detect ultraviolet light in a wavelength band that is harmful in particular to a human body.

The light receiving portion 2 may include two or more types of elements including an element that receives light having a wavelength of 200 nm or more and less than 220 nm and an element that receives light having a wavelength of 230 nm or more and less than 400 nm. In this case, designs of the element that receives ultraviolet light having a wavelength of 200 nm or more and less than 220 nm and the element that receives ultraviolet light having a wavelength of 230 nm or more and less than 400 nm may be changed. Thereby, it is possible to adopt an element with high photoelectric conversion efficiency or an inexpensive element with reduced cost in each wavelength band. Specifically, an ultraviolet sensor of an MSM type using AlGaN as a material can be used for an element that receives ultraviolet light having a wavelength of 200 nm or more and less than 220 nm, and a silicon photodiode can be used for an element that receives ultraviolet light having a wavelength of 230 nm or more and less than 400 nm.

The light receiving portion 2 may include two or more types of elements including an element that receives light having a wavelength of 230 nm or more and less than 240 nm and an element that receives light having a wavelength of 240 nm or more and less than 400 nm. In this case, designs of the element that receives ultraviolet light having a wavelength of 230 nm or more and less than 240 nm and the element that receives ultraviolet light having a wavelength of 240 nm or more and less than 400 nm may be changed. Thereby, it is possible to adopt an element with high photoelectric conversion efficiency or an inexpensive element with reduced cost in each wavelength band. Specifically, an ultraviolet sensor of an MSM type using AlGaN as a material can be used for an element that receives ultraviolet light having a wavelength of 230 nm or more and less than 240 nm, and a silicon photodiode can be used for an element that receives ultraviolet light having a wavelength of 240 nm or more and less than 400 nm.

The light receiving portion 2 may include two or more types of elements including an element that receives light having a wavelength of 220 nm or more and less than 230 nm and an element that receives light having a wavelength of 230 nm or more and less than 400 nm. In this case, designs of an element that receives ultraviolet light having a wavelength of 220 nm or more and less than 230 nm and an element that receives ultraviolet light having a wavelength of 230 nm or more and less than 400 nm may be changed. Further, in disposition for receiving light transmitting through the filter 3 to be described below, two or more types of elements including an element that receives light having a wavelength of 220 nm or more and less than 230 nm and an element that receives light having a wavelength of 230 nm or more and less than 400 nm may be disposed, or only the element that receives the light having a wavelength of 230 nm or more and less than 400 nm is disposed to receive light transmitting through the filter 3, and the element that receives the light having the wavelength of 220 nm or more and less than 230 nm may be disposed to receive light that does not pass through the filter 3. Thereby, it is possible to compare intensity of ultraviolet light having a wavelength of 220 nm or more and less than 230 nm with integrated output of ultraviolet light having a wavelength of 230 nm or more and less than 400 nm in each wavelength band. By comparing the integrated output, for example, a sterilization effect can be compared with an effect of harmfulness on a human body due to ultraviolet light. Furthermore, each of the sterilization effect and the effect of harmfulness on a human body due to ultraviolet light can also be quantified. The quantified value can be collated with various safety standards, and a drive current or voltage of the light source 4 can be controlled to be restricted to use within a range of safety.

In the light receiving portion 2, a response speed (time required for response) to ultraviolet light having a wavelength of 220 nm or more and less than 400 nm is preferably shorter than 1 second. When the response speed is shorter than 1 second, and when ultraviolet light having a wavelength of 230 nm or more and less than 400 nm, which is harmful to a human body, is emitted, it is possible to instantly notify surrounding persons of a risk. The response speed is more preferably shorter than 0.1 seconds, more preferably shorter than 0.01 seconds, and still more preferably shorter than 0.001 seconds. As the time is short, the time for notifying surroundings of a risk can be shortened.

Here, the "response speed" does not mean time until a signal is stabilized, but refers to time during which a change from a state without ultraviolet light can be detected, and a signal obtained from the light receiving portion 2 is not necessarily stabilized. Specifically, a method may be used in which a threshold that serves as a trigger is set and detection is performed when a detection signal exceeds a trigger setting value.

Further, in the light receiving portion 2, a response speed to ultraviolet light having a wavelength of 230 nm or more and less than 400 nm is preferably shorter than 0.1 seconds, more preferably shorter than 0.01 seconds, and still more preferably shorter than 0.001 seconds. As the time is short, time for notifying surroundings of safety can be shortened.

It is desirable that the light receiving portion 2 has a structure that receives light having a wavelength of 230 nm or more and less than 400 nm and converts the light into electricity (a current or electromotive voltage). Further, the light receiving portion 2 may have a structure in which a threshold of a current or electromotive voltage is set and a signal that can recognize surroundings is output when the current or electromotive voltage exceeds the threshold. The light receiving portion 2 may include two or more types of elements including an element that receives light having a wavelength of 220 nm or more and less than 230 nm and an element that receives light having a wavelength of 230 nm or more and less than 400 nm and have a structure in which respective elements sweep out separate electrical signals. In this case, by comparing integrated intensity of light having a wavelength of 220 nm or more and less than 230 nm with integrated intensity of light having a wavelength of 230 nm or more and less than 400 nm, a sterilization effect and an effect of harmfulness on a human body due to ultraviolet ray can be compared with each other. Furthermore, by comparing the above-described integrated intensities with each other, each of the sterilization effect and the effect of harmfulness on the human body due to ultraviolet ray can also be quantified. The quantified value can be collated with various safety standards, and a drive current or voltage of the light source 4 can be controlled to be restricted to use within a range of safety.

Light having a wavelength of 230 nm or more and less than 400 nm may be separated for each wavelength to measure a plurality of integrated intensities. For example, integrated intensity of light having a wavelength of 230 nm or more and less than 240 nm, integrated intensity of light having a wavelength of 240 nm or more and less than 250 nm, integrated intensity of light having a wavelength of 250 nm or more and less than 260 nm, integrated intensity of light having a wavelength of 260 nm or more and less than 270 nm, integrated intensity of light having a wavelength of 270 nm or more and less than 280 nm, and integrated intensity of light having a wavelength of 280 nm or more and less than 290 nm may be measured separately, and respective measurement results may be converted to control drive power of the light source 4.

This is particularly effective when there is wavelength dependence on an upper limit emission amount that serves as a reference for controlling input power to the light source 4. For example, in emitting light to a plant, an animal, or a resin product, the amount of influence on an emission target has wavelength dependence. Therefore, by measuring integrated intensity in each wavelength band as described above and by multiplying each integrated intensity of light by a weighting coefficient corresponding to the amount of influence in each wavelength band, even when light emission includes light intensity in different wavelengths, the integrated intensity can be compared to an upper reference limit. In one example, when an allowable upper limit emission amount of light emission with respect to integrated intensity of light having a wavelength of 260 nm or more and less than 270 nm is referred to as A, and when integrated intensity of light having a wavelength of 230 nm or more and less than 240 nm is referred to as B, a weighting coefficient thereof is referred to as b, integrated intensity of light having a wavelength of 240 nm or more and less than 250 nm is referred to as C, a weighting coefficient thereof is referred to as c, integrated intensity of light having a wavelength of 250 nm or more and less than 260 nm is referred to as D, a weighting coefficient thereof is referred to as d, integrated intensity of light having a wavelength of 260 nm or more and less than 270 nm is referred to as E, a weighting coefficient thereof is referred to as e, integrated intensity of light having a wavelength of 270 nm or more and less than 280 nm is referred to as F, a weighting coefficient thereof is referred to as f, and integrated intensity of light having a wavelength of 280 nm or more and less than 290 nm is referred to as G, and a weighting coefficient thereof is referred to as g, a method of adjusting (for example, reducing a current) input power to the light source 4 can be used such that B × b + C × c + D × d + E × e + F × f + G × g does not exceed A. In another example, when a light emission amount with respect to integrated intensity of light having a wavelength of 260 nm or more and less than 270 nm is referred to as A of a constant amount, and when integrated intensity of light having a wavelength of 230 nm or more and less than 240 nm is referred to as B, a weighting coefficient thereof is referred to as b, integrated intensity of light having a wavelength of 240 nm or more and less than 250 nm is referred to as C, a weighting coefficient thereof is referred to as c, integrated intensity of light having a wavelength of 250 nm or more and less than 260 nm is referred to as D, a weighting coefficient thereof is referred to as d, integrated intensity of light having a wavelength of 260 nm or more and less than 270 nm is referred to as E, a weighting coefficient thereof is referred to as e, integrated intensity of light having a wavelength of 270 nm or more and less than 280 nm is referred to as F, a weighting coefficient thereof is referred to as f, and integrated intensity of light having a wavelength of 280 nm or more and less than 290 nm is referred to as G, and a weighting coefficient thereof is referred to as g, a method of adjusting input power to the light source 4 such that B × b + C × c + D × d + E × e + F × f + G × g becomes A, for example, a method of increasing or decreasing a current can be used. In this case, a function to read in advance a calibration equation indicating a light emission output in each wavelength band with respect to a current and to perform calculation by using the calibration equation is a built-in the ultraviolet ray receiving and emitting device 5, and a method of adjusting a light emission amount with respect to integrated intensity of light having a wavelength of 260 nm or more and less than 270 nm to become A of a constant amount.

More specifically, there may be provided a function of emitting visible light to surroundings, emitting sound recognizable by a human body, performing vibration, displaying quantified data, or transmitting quantified data or a quantified signal when the threshold value is exceeded. Here, the "data" or "signal" may specifically indicate a value of a current or voltage converted when ultraviolet light is detected by the light receiving portion 2 or a value obtained by quantifying a ratio of a current or voltage, may be a value converted by multiplying the quantified value by a correction coefficient, or may represent division obtained by dividing the quantified value as a numerical value.

The light receiving portion 2 may include two or more types of elements including an element that receives light having a wavelength of 220 nm or more and less than 230 nm and an element that receives light having a wavelength less than 220 nm, and have a structure in which respective elements sweep out separate electrical signals. The light receiving portion 2 is an element that receives light having a wavelength less than 220 nm, thereby being able to detect whether the light having a wavelength less than 220 nm is emitted into the atmosphere, and as the light receiving portion 2 stops emitting light from the light source 4 according to the detection, generation of ozone generated by ultraviolet light in the same wavelength band can be suppressed.

A mechanism (hereinafter, referred to as a first light emission adjustment mechanism) that adjusts or suppresses input power to the light source 4 or light emission from the light source 4 by using a measurement intensity value measured by the light receiving portion 2 may be separately provided from the light receiving portion 2. For example, the measurement intensity value can include an integrated output intensity value in a certain wavelength band or a substitute value obtained by multiplying the integrated intensity value in a certain wavelength band by a weighting coefficient for each wavelength band. Alternatively, other than the integrated intensity value, an output intensity value at a certain wavelength or an electromotive voltage value generated at the time of receiving light can also be used. Alternatively, the light receiving portion 2 may have a built-in fluorescent substance and a built-in visible light sensor, the fluorescent substance may convert ultraviolet light into visible light, and the visible light sensor may calculate the fluorescence intensity of the visible light as a measurement intensity value. For example, the first light emission adjustment mechanism may detect an integrated intensity value of light having a wavelength of 220 nm or more and less than 230 nm and adjust input power to the light source 4 such that an output of light having a wavelength of 220 nm or more and less than 230 nm becomes a constant value. Further, although an output of the light source 4 decreases due to degradation of an element according to elapse of use time, in this case, the first light emission adjustment mechanism may adjust the input power to the light source 4 such that the same light emission intensity as an initial output is obtained. The first light emission adjustment mechanism may detect an integrated intensity value of light having a wavelength of 220 nm or more and less than 230 nm and adjust the input power to the light source 4 to 0 watts when the light emission intensity represents a constant relative ratio value (for example, 0.5 times) of the output at the beginning of use, or light emission from the light source 4 to the outside may be blocked by a mechanism, for example, closing a shutter, that mechanically suppresses emission of light. In this case, the first light emission adjustment mechanism may indicate that the light emission is suppressed by an indicator display in addition to a mechanical operation. The first light emission adjustment mechanism may measure intensity of each wavelength of light having a wavelength of 220 nm or more and less than 230 nm, and when a wavelength indicating a maximum output in this wavelength range is shifted by a specified value or more (for example, 2 nm), the input power to the light source 4 may be adjusted to 0 watts. Although the above describes an example of an operation in which the first light emission adjustment mechanism measures intensity of light having a wavelength of 220 nm or more and less than 230 nm, the first light emission adjustment mechanism may perform the same operation when the intensity of light having a wavelength of 230 nm or more and less than 320 nm is measured, or may perform the same adjustment when intensity of light having a wavelength of 220 nm or less is measured. Specifically, for example, when the intensity of light having a wavelength of 230 nm or more and less than 320 nm exceeds a specified value, the first light emission adjustment mechanism may adjust the input power to the light source 4 to be reduced, and thereby, the intensity of light having a wavelength of 220 nm or more and less than 320 nm is reduced.

A mechanism (hereinafter, referred to as a second light emission adjustment mechanism) that adjusts or suppresses input power to the light source 4 or light emission from the light source 4 by using a measurement intensity value of light having a wavelength less than 220 nm measured by the light receiving portion 2 may be separately provided from the light receiving portion 2. For example, the measurement intensity value can include an integrated output intensity value in a certain wavelength band less than 220 nm or a substitute value obtained by multiplying the integrated intensity value in a certain wavelength band by a weighting coefficient for each wavelength band. Alternatively, other than the integrated intensity value, an output intensity value at a certain wavelength or an electromotive voltage value generated at the time of receiving light can also be used. Alternatively, the light receiving portion 2 may have a built-in fluorescent substance and a built-in visible light sensor, the fluorescent substance may convert ultraviolet light into visible light, and the visible light sensor may calculate the fluorescence intensity of the visible light as a measurement intensity value. The second light emission adjustment mechanism may detect an integrated intensity value of light having a wavelength of 220 nm or more and less than 230 nm and adjust input power to the light source 4 to 0 watts when the light emission intensity represents a constant relative ratio value (for example, 0.5 times) of the output at the beginning of use, or light emission from the light source 4 to the outside may be blocked by a mechanism, for example, closing a shutter, that mechanically suppresses emission of light. In this case, the second light emission adjustment mechanism may indicate that light emission is suppressed by an indicator display in addition to a mechanical operation. The second light emission adjustment mechanism may measure intensity of each wavelength of light having a wavelength less than 220 nm, and when a wavelength indicating a maximum output in this wavelength range is shifted by a specified value or more (for example, 2 nm), the input power to the light source 4 may be adjusted to 0 watts. Alternatively, when the intensity of light having a wavelength less than 220 nm exceeds the specified value, the second light emission adjustment mechanism may adjust the input power to the light source 4 to be reduced, and thereby, the intensity of light having a wavelength less than 220 nm is reduced.

When the first light emission adjustment mechanism adjusts the input power to the light source 4 by using a signal from the light receiving portion 2, a voltage may be increased or decreased by using a step-up converter or a step-down converter, or may be controlled by a current-controlled resistor. Alternatively, a duty ratio or intensity of each pulse may be adjusted by pulse driving. When the duty ratio is changed, pulse time of each pulse may be changed or the number of pulses may be changed.

A signal detected by the light receiving portion 2 may be amplified by using a preamplifier, and the first light emission adjustment mechanism may send the amplified signal to a comparator and compare the amplified signal with a voltage for driving the light source 4. In this case, the first light emission adjustment mechanism may control both voltage values of a signal (voltage) amplified by using a preamplifier and a voltage for driving the light source 4 to be equal to each other, that is, the light source 4 may perform feedback-control such that a light emission output is constant. That is, the first light emission adjustment mechanism may perform a control such that, when an output voltage of the preamplifier is lower than a voltage for driving a light source, an output of a comparator increases and a light output increases as a drive current of the light source 4 increases, and may perform a control such that, when the output voltage of the preamplifier is higher than the voltage for driving the light source, the output of the comparator decreases, and the light output decreases as the drive current of the light source 4 decreases.

When cumulative time detected by the light receiving portion 2 exceeds a specified value (hour), the input power to the light source 4 from the light source 4 may be adjusted to 0 watts (that is, light emission is stopped), or a mechanism (hereinafter, referred to as a second light emission adjustment mechanism) for mechanically suppressing emission to the outside may be provided separately from the light receiving portion 2. By performing this operation, the second light emission adjustment mechanism can suppress emission of light harmful to a human body to the outside.

When a plurality of light sources 4 are provided, the second light emission adjustment mechanism may adjust input power to only some of the plurality of light sources 4 by using a signal value detected by the light receiving portion 2.

When there are a plurality of light sources 4, the light receiving portion 2 may detect light emission of only some of the plurality of light sources 4. For example, when the light source 4 includes 100 light emitting diodes, input power to the light source 4 may be adjusted to detect a light emission output of one of the light emitting diodes.

By detecting light emission from the light receiving portion 2 as described above and controlling the input power (a voltage, a current, or both the voltage and current) of the light source 4, a light emission output of the light source 4 can be kept constant during a use period. Alternatively, by detecting light emission by using the light receiving portion 2 and controlling the input power to the light source 4, the light emission output of the light source 4 can be controlled to be within a specified value, and the input power to the light source 4 can be controlled when the light emission output of the light source 4 is stronger or weaker than the specified value or deviates from the specified value.

The light receiving portion 2 may use a correction coefficient when calculating a signal. A correction coefficient used for light having a wavelength of 230 nm or more and less than 240 nm may be greater than a correction coefficient used for light having a wavelength of 220 nm or more and less than 230 nm. Furthermore, a correction coefficient used for light having a wavelength of 240 nm or more and less than 250 nm may be greater than a correction coefficient used for the light having a wavelength of 230 nm or more and less than 240 nm**.** Furthermore, a correction coefficient used for light having a wavelength of 250 nm or more and less than 280 nm may be greater than the correction coefficient used for light having a wavelength of 240 nm or more and less than 250 nm. Thereby, a signal can increase when ultraviolet ray in a wavelength band that is more harmful to a human body is detected, and thus, a risk can be recognized more quickly.

In this way, adjustment of a correction coefficient is more effective when one element is used to receive light.

Further, a correction coefficient used for light having a wavelength of 280 nm or more and less than 320 nm may be greater than a correction coefficient used for light having a wavelength of 320 nm or more and less than 400 nm. Thereby, a signal can increase when ultraviolet ray in a wavelength band that is more harmful to a human body is detected, and thus, a risk can be recognized more quickly.

In this way, adjustment of a correction coefficient is more effective when one element is used to receive light.

Further, a correction coefficient used for light having a wavelength of 200 nm or more and less than 220 nm may be greater than a correction coefficient used for light having a wavelength of 230 nm or more and less than 400 nm. Thereby, a signal for detecting ultraviolet ray in a wavelength band in which ozone is generated can increase, and thus, a risk can be recognized more quickly.

In the light receiving portion 2, light receiving sensitivity to light having a wavelength of 220 nm or more and less than 230 nm may be higher than light receiving sensitivity to light having a wavelength of 230 nm or more and less than 400 nm. Thereby, a signal for detecting ultraviolet ray in a wavelength band in which ozone is generated can increase, and thus, a risk can be recognized more quickly. More specifically, the light receiving sensitivity of the light having a wavelength of 220 nm or more and less than 230 nm may be 10 times higher than the light receiving sensitivity of the light having a wavelength of 230 nm or more and less than 400 nm**.**

The light receiving portion 2 may include a temperature sensor (not illustrated). The light receiving portion 2 includes a temperature sensor and is based on a signal thereof, temperature characteristics of an ultraviolet ray receiving device of the light receiving portion 2 are corrected, and more accurate measurement can be made. The temperature sensor may be an infrared sensor. When the temperature sensor is the infrared sensor, the temperature sensor converts infrared ray into an electrical signal and easily corrects temperature characteristics of an ultraviolet light receiving element. Further, when the infrared sensor is a semiconductor sensor, the ultraviolet light receiving element and an infrared ray receiving element may be disposed in parallel, and by disposing in parallel the infrared ray receiving element and the ultraviolet light receiving element in the same package product, it is also possible to realize space saving. Furthermore, a characteristic change due to exposure of an infrared sensor to ultraviolet ray can be corrected by performing processing thereof according to intensity detected by the ultraviolet light receiving element.

The light receiving portion 2 may measure an initial output of the light source 4, may measure an output thereof after a certain period of use, or may measure both. The light receiving portion 2 2 may measure an output of the light source 4 at all times, measure the output at regular intervals, or measure the output at a certain time. The light receiving portion 2 may perform measurement even when no light is emitted, such as when power of the light source 4 is off.

The light receiving portion 2 may receive light from the sun at the same time, but preferably, a partition or the like that blocks sunlight is provided so as not to directly receive light from the sun. Alternatively, the light receiving portion 2 may link timing of light emission from the light source 4 to timing of light reception of the light receiving portion 2 in order to reduce noise from sunlight. In this case, the light source 4 may be pulse-driven.

Hereinafter, each layer of the light receiving portion 2 is described in detail. Hereinafter, a case where a sensor of an MSM type using AlGaN for an ultraviolet light receiving sensor of the light receiving portion 2 is used is described.

### <Substrate>

In a specific example of a material forming the substrate 21, Si, SiC, MgO, GA₂O₃, Al₂O₃, ZnO, GaN, InN, AlN, mixed crystal thereof, or so on can be used. The substrate 21 preferably has a quadrangular shape of a thin plate but is not limited thereto.

Further, an off-angle of the substrate 21 is preferably larger than 0 degrees and smaller than 2 degrees from the viewpoint of growth of high-grade crystal.

A film thickness of the substrate 21 is not limited in particular when a purpose thereof is to stack an AlGaN thin film on an upper layer but is preferably 50 µm or more and 1 mm or less. The substrate 21 is used for the purpose of supporting an upper layer thin film, improving crystallinity, and dissipating heat to the outside. Therefore, an AlN substrate, which can be formed by growing AlGaN with high quality and is a material with high thermal conductivity, can be preferably used as the substrate 21.

Crystal quality of the substrate 21 is not limited in particular, but for the purpose of forming an element thin film with high photoelectric conversion efficiency on an upper layer, threading dislocation density is preferably 1×10⁹ cm⁻² or less, and more preferably 1×10⁵ cm⁻² or less. A growth surface of the substrate 21 may be +c-plane AlN which is generally used because of low cost but may be -c-plane AlN, a semi-polar plane substrate, or a non-polar plane substrate. When an AlN substrate is used as the substrate 21, the AlN substrate can be produced by a melting growth method, a sublimation method, or a halide vapor phase growth method, but the AlN substrate is preferably produced by the sublimation method from the viewpoint that a high quality AlN substrate can be grown.

### <Semiconductor stacked portion>

The semiconductor stacked portions 22, 23, and 24 are elements that convert ultraviolet light into electricity.

The semiconductor stacked portion 22 is disposed on the substrate 21 and is disposed for the purpose of growing the semiconductor stacked portions 23 and 24 which are stacked on an upper layer with high quality. Specifically, when a +c-plane AlN single crystal substrate is used as the substrate 21, the semiconductor stacked portion 22 is preferably AlGaN. This is because a thin film with high quality and a small lattice constant difference from the substrate 21 can be grown and ultraviolet light having a wavelength of 220 nm or more and less than 230 nm and ultraviolet light having a wavelength of 230 nm or more and less than 400 nm can be converted into electricity.

The semiconductor stacked portion 22 may have AlN disposed as a buffer layer (not illustrated) at an interface with the substrate 21. The buffer layer of AlN may be grown by the same method as the substrate 21 but may be grown by a different method. When the semiconductor stacked portion 22 is grown by a different method, the semiconductor stacked portion 22 may include impurities, such as carbon, oxygen, and hydrogen having concentrations different from a concentration of the substrate 21. When the semiconductor stacked portion 22 is grown by a different method, the semiconductor stacked portion 22 can be formed by an organic metal vapor phase growth method, a sputtering method, a halide vapor phase growth method, or the like, but the organic metal vapor phase growth method is preferable from the viewpoint that a high quality AlGaN layer of an upper layer can be continuously grown.

An AlN buffer layer may be disposed in an upper layer of the substrate 21, specifically, a region in contact with the substrate 21 in a film thickness direction of the semiconductor stacked portion 22. The AlN buffer layer is formed on, for example, an entire surface of the substrate 21. A film thickness of the AlN buffer layer is preferably more than 10 nm and less than 10 µm. As the film thickness of the AlN buffer layer is more than 10 nm, the AlN with high crystallinity can be formed. Further, when the film thickness of the AlN buffer layer is less than 10 µm, crystal growth without cracks can be made on an entire surface of a wafer. The film thickness of the AlN buffer layer is more preferably more than 50 nm and less than 5 µm. As the film thickness of the AlN buffer layer is more than 50 nm, the AlN with high crystallinity can be formed with high reproducibility. Further, when the film thickness of the AlN buffer layer is less than 5 µm, crystal growth with low crack occurrence probability can be made.

When the substrate 21 is formed of a nitride semiconductor, such as GaN, AlN, and AlGaN, a nitride semiconductor layer with few defects can be grown on the substrate 21 for the above-described reason. Therefore, when the substrate 21 is formed of a nitride semiconductor, the AlN buffer layer does not necessarily have to be provided. Further, also on another upper portion of the substrate, high-quality AlGaN may be formed directly on the substrate and does not need to have AlN. An AlN layer may be mixed with impurities, such as carbon, silicon, iron, and magnesium.

The semiconductor stacked portions 23 and 24 are preferably formed of AlGaN with different Al composition ratios from the viewpoint of generating two-dimensional electron gas at an interface therebetween. The semiconductor stacked portions 23 and 24 configure a mesa structure 20.

Specifically, when the substrate 21 is a +c-plane AlN substrate, the semiconductor stacked portion 23 is Al_{0.5}Ga_{0.5}N having a film thickness of 150 nm, and the semiconductor stacked portion 24 is Al_{0.6}Ga_{0.4}N having a film thickness of 20 nm, an ultraviolet light receiving element with spectral sensitivity of 1000 A/W/nm or more in a wavelength band of a wavelength of 220 nm or more and less than 230 nm and spectral sensitivity of 3 A/W/nm or more in a wavelength band of a wavelength of 230 nm or more and less than 270 nm can be formed.

When the substrate 21 is a +c-plane AlN substrate, the semiconductor stacked portion 23 is Al_{0.2}Ga_{0.8}N having a film thickness of 150 nm, and the semiconductor stacked portion 24 is GaN having a film thickness of 20 nm, an ultraviolet light receiving element with spectral sensitivity of 10000 A/W/nm or more in a wavelength band of a wavelength of 220 nm or more and less than 230 nm and spectral sensitivity of 3 A/W/nm or more in a wavelength band of a wavelength of 230 nm or more and less than 4000 nm can be formed.

Al composition ratios of the semiconductor stacked portions 22, 23, and 24 can be specified by an energy dispersive X-ray spectroscopy (EDX) of a cross-sectional structure.

A cross section is exposed along an a-plane of AlGaN by using a focused Ion beam (FIB) device. A transmission electron microscope is used as a method of observing the cross section. A magnification of observation is x1000 times/nm with respect to a film thickness of a layer to be measured. For example, a layer having a film thickness of 100 nm is observed at a magnification of 100,000 times, and a layer having a film thickness of 1 µm is observed at a magnification of 1,000,000 times.

An Al composition ratio can be defined as a ratio of the number of molecules of Al to the sum of the number of molecules of Al and the number of molecules of Ga, and specifically, the Al composition ratio can be defined by using a value of the number of molecules of Al and Ga analyzed and quantified from EDX.

Further, V-type elements including P, As, and Sb other than N, and impurities, such as C, H, F, O, Mg, and Si may be included in Alₓ₁Ga₍₁₋ₓ₁₎N forming the semiconductor stacked portion 22, 23, and 24. The semiconductor stacked portions 22, 23, and 24 may be n-type semiconductors or p-type semiconductors.

When the semiconductor stacked portions 22, 23, and 24 are formed of n-type semiconductors, AlGaN can be made into an n-type semiconductor by being doped (for example, 1×10¹⁹ cm⁻³) with, for example, Si.

When the semiconductor stacked portions 22, 23, and 24 are formed of p-type semiconductors, AlGaN can be made into a p-type semiconductor, be being doped (for example, 3×10¹⁹ cm⁻³) with, for example, Mg.

A different layer other than the above-described layer may be included. For example, an intermediate AlGaN layer (not illustrated) may be included between the semiconductor stacked portion 22 and the semiconductor stacked portion 23. The intermediate AlGaN layer may be grown for the purpose of reducing lattice distortion of the semiconductor stacked portions 23 and 24 stacked on an upper layer, or a film thickness thereof may be grown to improve crystallinity.

In FIG. 2, the semiconductor stacked portions 23 and 24 have a structure having a smaller area than the semiconductor stacked portion 22. Specifically, the semiconductor stacked portions 23 and 24 are formed by performing etching treatment after thin films of the semiconductor stacked portions 22, 23, and 24 are grown. The treatment obtains a detection signal for ultraviolet light with high reproducibility by controlling a light-receiving area or is performed for the purpose of suppressing adhesion of particles during element isolation.

### <Electrode>

The electrodes 25 and 26 are preferably formed of a material that has an ohmic connection with the semiconductor stacked portion 24 that is in contact therewith. A material forming the electrodes 25 and 26 includes Ti, Al, Ni, Au, Cr, V, Zr, Hf, Nb, Ta, Mo, W, an alloy thereof, ITO, or the like, and a material including aluminum and nickel or a material including Zr, V, Al, Mo, and Au is more preferable from the viewpoint of reducing contact resistance.

The electrodes 25 and 26 may be formed by continuously forming metal layers, such as Au, Al, Cu, Ag, or W for current diffusion or connection with a package substrate (not illustrated), and it may be desirable to use Au with high conductivity therefor. Further, in order to improve adhesion, Ti may be further included in an interface with the semiconductor stacked portion 24.

### <Filter>

The filter 3 is disposed to control a wavelength of ultraviolet light incident on the light receiving portion 2, and specifically, attenuates light having a wavelength of 220 nm or more and less than 230 nm. The ultraviolet light having the wavelength of 220 nm or more and less than 230 nm is not harmful to a human body, thereby being positively used, and is present at a higher light intensity than ultraviolet light having a wavelength of 200 nm or more and less than 220 nm or ultraviolet light having a wavelength of 230 nm or more and less than 400 nm. As the filter attenuates ultraviolet light having a wavelength of 220 nm or more and less than 230 nm, the light receiving portion 2 can accurately detecting the ultraviolet light having the wavelength of 230 nm or more and less than 400 nm. Here, the "attenuation" does not necessarily mean that transmissivity is 0%, and when the transmissivity is 30% or less in at least the entire designated wavelength range, it is considered that attenuation is made.

There may be one filter 3 or a plurality of filters 3. Where there are the plurality of filters 3, the plurality of filters 3 may have different spectral characteristics.

The filter 3 transmits light in a wavelength band of a wavelength of 230 nm or more and less than 320 nm therethrough. Here, "transmit" means that transmissivity is higher in the entire designated wavelength range than light in a wavelength band of a wavelength of 220 nm or more and less than 230 nm, which is "attenuated". In the filter 3, transmissivity of light having a wavelength of 220 nm or more and less than 230 nm is preferably one-tenth or less of transmissivity of light having a wavelength of 230 nm or more and less than 320 nm. Here, in the filter 3, transmissivity of light having a wavelength of 220 nm or more and less than 230 nm is preferably one-tenth or less of transmissivity of light having a wavelength of 230 nm or more and less than 240 nm, further preferably one-tenth or less of transmissivity of light having a wavelength of 240 nm or more and less than 250 nm, further preferably one-tenth or less of transmissivity of light having a wavelength of 250 nm or more and less than 260 nm, further preferably one-tenth or less of transmissivity of light having a wavelength of 260 nm or more and less than 270 nm, further preferably one-tenth or less of transmissivity of light having a wavelength of 270 nm or more and less than 280 nm, further preferably one-tenth or less of transmissivity of light having a wavelength of 280 nm or more and less than 290 nm, further preferably one-tenth or less of transmissivity of light having a wavelength of 290 nm or more and less than 300 nm, further preferably one-tenth or less of transmissivity of light having a wavelength of 300 nm or more and less than 310 nm, and further preferably one-tenth or less of transmissivity of light having a wavelength of 310 nm or more and less than 320 nm. Furthermore, it is more preferably one-tenth or less of a transmissivity of light having a wavelength of 230 nm or more and less than 400 nm. Here, in the filter 3, transmissivity of light having a wavelength of 220 nm or more and less than 230 nm is preferably one-tenth or less of transmissivity of light having a wavelength of 320 nm or more and less than 330 nm, further preferably one-tenth or less of transmissivity of light having a wavelength of 330 nm or more and less than 340 nm, further preferably one-tenth or less of transmissivity of light having a wavelength of 340 nm or more and less than 350 nm, further preferably one-tenth or less of transmissivity of light having a wavelength of 350 nm or more and less than 360 nm, further preferably one-tenth or less of transmissivity of light having a wavelength of 360 nm or more and less than 370 nm, further preferably one-tenth or less of transmissivity of light having a wavelength of 370 nm or more and less than 380 nm, further preferably one-tenth or less of transmissivity of light having a wavelength of 380 nm or more and less than 390 nm, and further preferably one-tenth or less of transmissivity of light having a wavelength of 390 nm or more and less than 400 nm. Thereby, since intensity of ultraviolet light having a wavelength of 220 nm or more and less than 230 nm which is incident on the light receiving portion 2 as described above is reduced, ultraviolet light having a wavelength of 230 nm or more and less than 320 nm or ultraviolet light having a wavelength of 230 nm or more and less than 400 nm can be more efficiently received and detected.

The filter 3 preferably has a higher transmissivity in the order of light having a wavelength of 220 nm or more and less than 230 nm, light having a wavelength of 230 nm or more and less than 240 nm, and light having a wavelength of 240 nm or more and less than 250 nm. Ultraviolet light is more harmful to a human body in this order. Therefore, it is preferable that transmissivity of the filter 3 increases in this order such that the light receiving portion 2 can detect ultraviolet light in a more harmful wavelength band.

Further, transmissivity of light having a wavelength of 230 nm or more and less than 240 nm is preferably higher than transmissivity of light having a wavelength of 220 nm or more and less than 230 nm. Furthermore, transmissivity of light having a wavelength of 250 nm or more and less than 240 nm is preferably higher than the transmissivity of light having the wavelength of 230 nm or more and less than 240 nm. Furthermore, transmissivity of light having a wavelength of 250 nm or more and less than 280 nm is preferably higher than the transmissivity of light having the wavelength of 250 nm or more and less than 240 nm. Thereby, the light receiving portion 2 can detect more efficiently ultraviolet light in a wavelength band that is harmful to a human body. This is more effective when there is one filter 3. Further, the filter 3 preferably has a higher transmissivity of light having a wavelength of 320 nm or more and less than 400 nm than transmissivity of light having a wavelength of 280 nm or more and less than 320 nm. The transmissivity of light having the wavelength of 280 nm or more and less than 320 nm is preferably higher than transmissivity of light having a wavelength of 220 nm or more and less than 230 nm. The transmissivity of light having the wavelength of 320 nm or more and less than 400 nm is preferably higher than the transmissivity of light having the wavelength of 220 nm or more and less than 230 nm.

In the filter 3, transmissivity of light having a wavelength of 220 nm or more and less than 230 nm is more preferably 1% or less, and still more preferably 0.1% or less. This may be satisfied by only a part of the wavelength band of 220 nm or more and less than 230 nm, or by the entire wavelength band. Thereby, the light receiving portion 2 can more efficiently detect ultraviolet light in a wavelength band that is harmful to a human body.

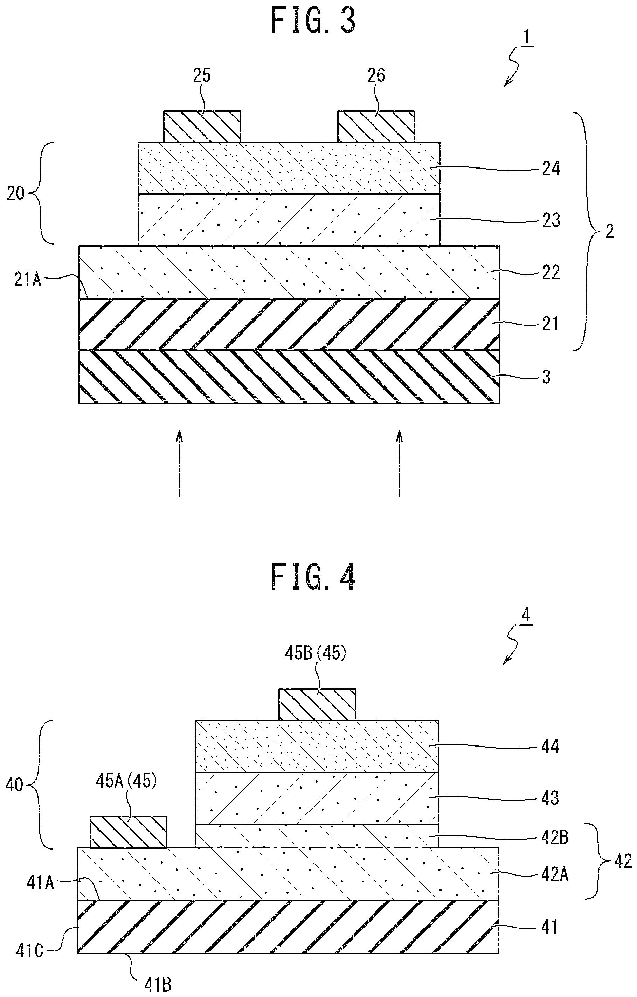

When the light receiving portion 2 includes a semiconductor light receiving element, the filter 3 may be disposed in contact with an incident surface of the semiconductor light receiving element. Specifically, each layer of the ultraviolet ray receiving device 1 may be disposed as illustrated in FIG. 3. Particularly, when the light receiving portion 2 includes a photodiode as the semiconductor light receiving element, the filter 3 is preferably disposed in contact with the semiconductor light receiving element, that is, a surface (incident surface) of the photodiode. Because the filter 3 is disposed in contact with the incident surface of the semiconductor light receiving element, functions of the filter 3 and the semiconductor light receiving element are integrated, and thus, costs can be reduced. Furthermore, when the filter 3 covers a surface of the semiconductor light receiving element, corrosion of the semiconductor light receiving element by air or water can be suppressed.

The filter 3 may transmit light having a wavelength of 200 nm or more and less than 220 nm therethrough, and the light receiving portion 2 may receive the light having the wavelength of 200 nm or more and less than 220 nm. Thereby, the light receiving portion 2 can receive ultraviolet light having a wavelength of 200 nm or more and less than 220 nm, which generates ozone.

The filter 3 may have transmissivity of the light having the wavelength of 200 nm or more and less than 220 nm higher than transmissivity of light having a wavelength of 230 nm or more and less than 240 nm. Thereby, ultraviolet light that generates ozone can be detected preferentially and more efficiently than ultraviolet light having a wavelength of 230 nm or more and less than 240 nm, which is directly harmful to an exposed human body. This is because, for example, direct emission of ultraviolet light can be blocked to some extent in a space partitioned by partitions or the like, but ultraviolet light in a wavelength band that generates ozone gas can be effectively detected in an environment where there is a concern about diffusion of the ozone gas.

For example, a dielectric multilayer film is used for the filter 3 from the viewpoint of ease of optical design. For example, a filter can be formed of a dielectric multilayer film in which an SiO₂ layer having a thickness of 65 nm and an HfO₂ layer having a thickness of 10 nm are stacked repeatedly for five cycles, and the filter has a high blocking rate in an ultraviolet wavelength band of a wavelength of 222 nm to 235 nm. By appropriately designing a material, a film thickness, and a cycle of the dielectric multilayer film, a band of a wavelength to be blocked and transmissivity in each wavelength band can be controlled. The dielectric multilayer film can be formed by, for example, a sputtering method. The dielectric multilayer film may be formed of SiO₂, Al₂O₃, SiN, SnO₂, ZrO, HfO₂, or the like.

### (1.3) Light source

The light source 4 may emit both light having a wavelength of 220 nm or more and less than 230 nm and light having a wavelength of 230 nm or more and less than 400 nm. In this case, ultraviolet light having a wavelength of 230 nm or more and less than 400 nm is harmful to a human body but can be detected by the light receiving portion 2 of the present disclosure. The ultraviolet ray receiving and emitting device 5 of the present disclosure can drive the light source 4 while checking that emission of ultraviolet light having a wavelength of 230 nm or more and less than 400 nm from the light source 4 does not exceed an allowable threshold output. Thereby, it is not necessary to limit the emission of the light source 4 to light having a wavelength of 220 nm or more and less than 230 nm, and thus, design and manufacture of the light source 4 can be easily performed and manufacturing costs can be reduced.

Light emission intensity of light having a wavelength of 220 nm or more and less than 230 nm from the light source 4 may be 10 times or more the light emission intensity of light having a wavelength of 230 nm or more and less than 400 nm. By using the ultraviolet ray receiving and emitting device 5, intensity of ultraviolet light having a wavelength of 220 nm or more and less than 230 nm can be increased within an allowable range of a wavelength of 230 nm or more and less than 400 nm, which is harmful to a human body.

The light emission intensity of light having a wavelength of 220 nm or more and less than 230 nm from the light source 4 may be 10 times or more the light emission intensity of light having a wavelength of 200 nm or more and less than 220 nm. By using the ultraviolet ray receiving and emitting device 5, intensity of ultraviolet light having a wavelength of 220 nm or more and less than 230 nm can be increased within an allowable range of a wavelength of 200 nm or more and less than 220 nm, which is harmful to a human body.

An output of light having a wavelength of 200 nm or more and less than 220 nm from the light source 4 may be higher than an output of light having a wavelength of 230 nm or more and less than 240 nm. In an environment in which light is continuously emitted from the light source 4, ozone generated by ultraviolet light having a wavelength of 200 nm or more and less than 220 nm can be decomposed by ultraviolet light having a wavelength of 230 nm or more and less than 240 nm and can increase an allowable threshold output of ultraviolet light having a wavelength of 200 nm or more and less than 220 nm.

Thereby, a degree of freedom in designing the light source 4 can be increased, and costs can be reduced.

Further, the light receiving portion 2 may be disposed at a position where the light receiving portion 2 receives light having intensity of one-hundredth or less of the total emission from the light source 4. Thereby, it is possible to efficiently emit and use emitted light of ultraviolet ray from the light source 4, and ultraviolet light in a wavelength band that is harmful to a human body can be detected.

The light source 4 may be an excimer lamp, a light emitting diode (LED), a laser diode, or an ultraviolet laser. The LED is preferable from the viewpoint of low cost, long life, robustness, and less environmental pollution at the time of disposition. The LED is preferable to use AlGaN as a material. By using AlGaN as a material, the light source 4 can be provided which does not emit ultraviolet light having a wavelength of 210 nm or less that generates an extremely large amount of ozone. When the light source 4 is an LED, the light source 4 includes a form sealed with a resin or the like, a form mounted on a sub-mount substrate, and the like.

The light source 4 may emit light having a wavelength of 220 nm or more and less than 240 nm. By allowing emission of light having a wavelength of 220 nm or more and less than 240 nm, particularly when an LED is used as the light source 4, a degree of freedom in a structural design of an active layer is increased, and thus, costs can be reduced.

The light source 4 may emit light having a wavelength of 220 nm or more and less than 250 nm. By allowing emission of light having a wavelength of 220 nm or more and less than 250 nm, particularly when an LED is used as the light source 4, a degree of freedom in a structural design of a cladding layer sandwiching an active layer is increased, and thus, costs can be reduced. Specifically, for example, AlGaN with a lower Al composition ratio than an Al composition ratio of an active layer can be used as a p-type semiconductor, and thus, element resistance and a drive voltage can be reduced.

When the light source 4 is an LED, the ultraviolet ray receiving and emitting device 5 includes, for example, an LED substrate 41, a first conductivity type semiconductor layer 42, an active layer 43, a second conductivity type semiconductor layer 44, and an LED electrode 45. That is, it is considered that the ultraviolet ray receiving and emitting device 5 has a structure in which a light emitting element formed of an AlGaN material is formed on the LED substrate 41. In the ultraviolet ray receiving and emitting device 5, the first conductivity type semiconductor layer 42 and the second conductivity type semiconductor layer 44 having the active layer 43 sandwiched therebetween function as a cladding layer.

The light source 4 may emit light having a wavelength of 220 nm or more and less than 230 nm and light having a wavelength of 260 nm or more and less than 280 nm. By allowing emission of light having a wavelength of 260 nm or more and less than 280 nm, particularly when an LED is used as the light source 4, a degree of freedom in a structural design of a cladding layer is further increased, and thus, costs can be reduced. Specifically, AlGaN with a lower Al composition ratio can be used as, for example, a p-type semiconductor, and thus, element resistance can be further reduced and a drive voltage can be reduced.

Further, because an Al composition ratio can be further reduced, for example, when a composition gradient structure in which an Al composition ratio of AlGaN is continuously reduced in a direction away from the active layer 43 is used as a cladding layer, a change rate of the Al composition ratio can be increased. Therefore, because the amount of holes generated by a polarity-doping method can be increased, element resistance can be reduced and a drive voltage can be reduced. Alternatively, carrier injection efficiency can be improved to increase light emission efficiency of an LED.

The light source 4 may emit light having a wavelength of 220 nm or more and less than 230 nm and light having a wavelength of 280 nm or more and less than 400 nm. By allowing emission of light having a wavelength of 280 nm or more and less than 400 nm, when an LED is used as the light source 4, a degree of freedom in a structural design of a cladding layer is increased, and thus, costs can be reduced. Specifically, for example, GaN can be used as a p-type semiconductor, and thus, for example, when a composition gradient structure in which an Al composition ratio of AlGaN is continuously reduced in a direction away from the active layer 43 is used as a cladding layer, a change rate of the Al composition ratio can be increased. Therefore, because the amount of holes generated by a polarity-doping method can be increased, element resistance can be reduced and a drive voltage can be reduced. Alternatively, carrier injection efficiency can be improved to increase light emission efficiency of an LED.

The light source 4 may emit light having a wavelength of 220 nm or more and less than 230 nm and light having a wavelength of 200 nm or more and less than 220 nm. By allowing emission of light having a wavelength of 200 nm or more and less than 220 nm, when an LED is used as the light source 4, manufacturing tolerance in an Al composition ratio of AlGaN used as the active layer 43 can be allowed, and manufacturing costs can be reduced. Further, by using the ultraviolet ray receiving and emitting device 5, the ultraviolet ray receiving and emitting device 5 can be used while checking that ultraviolet light having a wavelength of 200 nm or more and less than 220 nm is within an allowable emission range.

The ultraviolet ray receiving and emitting device 5 may include a circuit that stops driving of the light source 4 when a detection value of the light receiving portion 2 exceeds a threshold. Thereby, it is possible to stop emission of ultraviolet light which is output from the light source 4 and is harmful to a human body. Alternatively, the ultraviolet ray receiving and emitting device 5 may have a circuit that changes a drive current (or voltage) of the light source 4 according to a detection value of the light receiving portion 2. Thereby, the ultraviolet ray receiving and emitting device 5 can emit light with a stable output or can suppress emission of ultraviolet light that is harmful to a human body from the light source 4 in excess of an allowable amount.

The ultraviolet ray receiving and emitting device 5 may have a structure in which a light emission output of the light source 4 is changed according to a detection value of the light receiving portion 2. For example, by disposing a window that controls an emission range of ultraviolet light at an emission portion of the light source 4 and controlling an output by changing a size of an opening of the window, it is possible to suppress emission of ultraviolet light that is harmful to a human body from the light source 4 in excess of an allowable amount. The ultraviolet ray receiving and emitting device 5 may have a structure in which emission to the external world is blocked by completely closing a window.

The ultraviolet ray receiving and emitting device 5 may have a mechanism (hereinafter, referred to as a third light emission adjustment mechanism) in which light is emitted from the light source 4 to the outside when a detection value of the light receiving portion 2 exceeds a threshold. For example, the ultraviolet ray receiving and emitting device 5 is confined in a closed space, and the third light emission adjustment mechanism opens the window for light emission and emits light to the outside when a detection value of the light receiving portion 2 exceeds the threshold. Thereby, the third light emission adjustment mechanism can check that ultraviolet light which is harmful to a human body is not emitted from the light source 4 and then emit the light from the light source 4 to the outside.

The ultraviolet ray receiving and emitting device 5 may include a temperature sensor or may include a temperature correction circuit that corrects an output of the light source 4 according to a temperature change. Thereby, it is possible to suppress a light emission output from changing due to temperature characteristics of the light source 4 whenever temperature changes.

The ultraviolet ray receiving and emitting device 5 may include a sensor that receives visible light having a wavelength of 400 nm or more and less than 800 nm. In a case where the light source 4 is an LED, when the LED is degraded with use, defects of the LED increase, and intensity of visible light emitted through the defects increases. By detecting the intensity of the visible light by using the sensor that receives the visible light, failure of the LED can be detected early.
Thereby, failure of a device can be detected early, and replacement time can be determined.

The ultraviolet ray receiving and emitting device 5 may be disposed such that a light emitting surface (surface on the LED substrate 41 side) of the ultraviolet ray receiving and emitting device 5 faces a light receiving surface (surface on the LED substrate 21 side) of the light receiving portion 2. The light emitting surface of the ultraviolet ray receiving and emitting device 5 may not face the light receiving surface 2 of the light receiving portion 2, but it is preferable that the light emitting surface does not face the light receiving surface from the viewpoint of efficiently emitting light from the light source 4 to the outside.

Further, the ultraviolet ray receiving and emitting device 5 may be disposed at a position where an angle formed by a perpendicular line of the light receiving surface of the light receiving portion 2 and a perpendicular line of the light emitting surface of the light source 4 is 45 degrees or more and less than 90 degrees. With this structure, for example, in a case where an LED formed of an AlGaN material is used as the light source 4, when the ultraviolet ray receiving and emitting device 5 and the light receiving portion 2 are disposed at a position where an angle formed by a perpendicular line of a light emitting surface is 45 degrees or more and less than 90 degrees, ultraviolet light that is harmful to a human body can be easily detected. This is because a ratio of light emission intensity of ultraviolet light having a wavelength of 230 nm or more and less than 400 nm to light emission intensity of ultraviolet light having a wavelength of 220 nm or more and less than 230 nm is increased, and thus, light having a wavelength of 230 nm or more and less than 400 nm, which is ultraviolet light that is harmful to a human body, can be easily detected.

The ultraviolet ray receiving and emitting device 5 may have a monolithic structure in which the light receiving portion 2 and the light source 4 are formed of the same semiconductor element. Specifically, a light receiving element having an AlGaN PIN structure and a light emitting element having an AlGaN PIN structure may be formed on the LED substrate 41. As the light receiving portion 2 and the light source 4 have a monolithic structure, manufacturing costs can be reduced.

Further, when the light source 4 is an LED, a transmission control unit that is a filter may be disposed on a back surface of the LED substrate 41.

Hereinafter, each layer of the ultraviolet ray receiving and emitting device 5 when the light source 4 is an LED is described with reference to FIG. 4. Hereinafter, the light source 4 is referred to as an LED 4.

### (1.3.1) Configuration of LED

The LED 4, which is an example of the light source 4 of the present embodiment, is a semiconductor element capable of emitting ultraviolet light.

The LED 4 includes the LED substrate 41, an LED thin film 40, and the LED electrode 45. Further, the LED thin film 40 is configured with the first conductivity type semiconductor layer 42, the active layer 43, and the second conductivity type semiconductor layer 44. The LED 4 includes a form sealed with a resin or the like, a form mounted on a sub-mount substrate, and the like.

Hereinafter, each layer is described in detail.

### <LED substrate>

The LED thin film 40 may be disposed on a first main surface 41A of the LED substrate 41 and a shape thereof is not limited in particular.

From the viewpoint of disposing the LED thin film 40 with high quality, the LED substrate 41 is preferably a sapphire substrate or an aluminum nitride substrate. The aluminum nitride substrate is preferably a single-crystal aluminum nitride substrate.

In a case where the other of the first main surface 41A of the LED substrate 41 is exposed to the external world of the LED 4, when the LED substrate 41 is a sapphire substrate, the LED substrate 41 can react with water or the like in an external world due to heat generated by the LED 4 to form a hydroxide film on a surface of the LED substrate 41. Further, when the LED substrate 41 is an aluminum nitride substrate, the LED substrate 41 can react with oxygen in the external world due to the heat generated by the LED 4 to form an oxide film. This film (the hydroxide film or the oxide film) attenuates or reflect light including a central wavelength of a light emission spectrum, thereby reducing light emission efficiency of light having a desirable wavelength, that is, reducing an output. This film may include carbon dioxide in the external world at the time of formation, carbon mixed as an impurity in the LED substrate 41, or carbon derived from a resin used in a package of the LED 4. Thereby, even when bandgap energy of each film is larger than light emission energy, absorption derived from impurities occurs and light is attenuated. Further, when layers having different refractive indices are formed, reflection occurs at an interface with the LED substrate 41. Particularly, when a material with a refractive index lower than a refractive index of the LED substrate 41 is formed as in, for example, an aluminum nitride substrate and an aluminum oxide layer, stronger reflection occurs and light cannot be emitted to the external world.

Dislocation density of the LED substrate 41 is preferably less than 10⁷ cm⁻², and more preferably less than 10⁵ cm⁻². From the viewpoint of reducing the dislocation density of the LED thin film 40, a root mean square (RMS) height Rq on the first main surface 41A side of the LED substrate 41 is preferably less than about 1 nm with respect to an area of 10 µm × 10 µm. Further, in order to form a flat and even transmission control unit, the root mean square (RMS) height on a second main surface 41B side of the LED substrate 41 is preferably less than about 10 nm with respect to an area of 10 µm × 10 µm.

### <LED thin film>

The LED thin film 40 includes the active layer 43 and is disposed on the first main surface 41A of the LED substrate 41 but is not limited in particular.

From the viewpoint of light emission efficiency, the LED thin film 40 may further include the first conductivity type semiconductor layer 42 and the second conductivity type semiconductor layer 44 so as to have the active layer 43 sandwiched therebetween. Here, a "first conductivity type" and a "second conductivity type" mean that semiconductors have different conductivities from each other, and when one semiconductor has n-type conductivity, the other semiconductor has p-type conductivity. Generally, a space between the active layer 43 and the LED substrate 41 is an n-type semiconductor layer but is not limited thereto.

At least one of spaces between layers other than the first conductivity type semiconductor layer 42, the active layer 43, and the second conductivity type semiconductor layer 44 are, for example, at least one of spaces between the active layer 43, the first conductivity type semiconductor layer 42 and the second conductivity type semiconductor layer 44 may include a layer (not illustrated) for blocking electrons or holes.

Further, from the viewpoint of improving crystallinity of the LED thin film 40, a buffer layer (not illustrated) may be preferably further provided on a surface of the LED thin film 40 in contact with the LED substrate 41.

Further, from the viewpoint of efficiently supplying power to the active layer 43, the LED electrode 45 (LED electrode 45A) may be provided to be in contact with the first conductivity type semiconductor layer 42, and the LED electrode 45 (LED electrode 45B) may be provided to be in contact with the second conductivity type semiconductor layer 44.

Further, the LED thin film 40 may have a mesa structure in which the active layer 43 is partially included. In FIG. 4, the mesa structure is configured by a second stacked region 42B of the first conductivity type semiconductor layer 42, the active layer 43, and the second conductivity type semiconductor layer 44.

The LED thin film 40 can be formed by, for example, a metal organic chemical vapor deposition (MOCVD) method. The LED thin film 40 having the mesa structure can be formed by forming a thin film layer constituting the LED thin film 40 by using the above-described MOCVD method or the like and then etching a desirable region.

### (Active layer)

The active layer 43 emits light corresponding to a band gap of the active layer 43 when power is applied to the active layer 43. The active layer 43 in the LED 4 of the present embodiment is not limited in particular as long as light having a central wavelength of a light emission spectrum of 220 nm or more and less than 230 nm is emitted. Here, when the light emission spectrum has a plurality of peaks, a wavelength of the peak with the highest light emission intensity is defined as a central wavelength of light.

For example, a quantum well structure can be used as a specific structure of the active layer 43. For example, a quantum well structure in which AlGaN layers with different composition ratios (different bandgaps) are stacked can be adopted. More preferably, a multiple quantum well obtained by stacking multiple AlGaN layers with different composition ratios (different bandgaps) can be adopted. More specifically, a triple quantum well structure can be used in which three well layers (having a thickness of 1 nm) with composition of Al_{0.87}Ga_{0.13}N and two barrier layers (having a thickness of 5 nm) with composition of Al_{0. 97}Ga_{0. 03}N are alternately stacked.

### (First conductivity type semiconductor layer)

Single crystal and mixed crystal of AlN, GaN, and InN can be used as the first conductivity type semiconductor layer 42, and a combination (multiple layers) thereof may be used. When the LED substrate 41 is an aluminum nitride substrate, AlGaN with a small difference in lattice constant and an Al/(Al+Ga) ratio of 0.8 or more can be preferably used as the first conductivity type semiconductor layer 42.

As illustrated in FIG. 4, the first conductivity type semiconductor layer 42 includes a first stacked region 42A formed by removing a part of the first conductivity type semiconductor layer 42 and a second stacked region 42B that is located on the first stacked region 42A and has a mesa structure.

When a first conductivity type semiconductor constituting the first conductivity type semiconductor layer 42 is an n-type semiconductor, for example, AlGaN doped with Si at a concentration of 1×10¹⁹ cm⁻³ can be used as the first conductivity type semiconductor. Further, n-type AlGaN made by a polarity-doping method that continuously changes a mixed crystal composition ratio of a mixed crystal semiconductor having polarity may be used as the first conductivity type semiconductor.

When the first conductivity type semiconductor constituting the first conductivity type semiconductor layer 42 is a p-type semiconductor, for example, AlGaN doped with Mg at a concentration of 3×10¹⁹ cm⁻³ can be used as the first conductivity type semiconductor. Further, p-type AlGaN made by a polarization doping method that continuously changes a mixed crystal composition ratio of a mixed crystal semiconductor having polarity may be used as the first conductivity type semiconductor. From the viewpoint of efficiently transferring carriers to an active layer 43 that emits light having a peak wavelength less than 230 nm, AlGaN with an Al/(Al+Ga) ratio of 0.8 or more is preferably used as the first conductivity type semiconductor.

### (Second conductivity type semiconductor layer)

Single crystal and mixed crystal of AlN, GaN, and InN can be used as the second conductivity type semiconductor layer 44, and a combination (multiple layers) thereof may be used.

When a second conductivity type semiconductor constituting the second conductivity type semiconductor layer 44 is an n-type semiconductor, for example, AlGaN doped with Si at a concentration of 1×10¹⁹ cm⁻³ can be used as the second conductivity type semiconductor. Further, n-type AlGaN made by a polarization doping method that continuously changes a mixed crystal composition ratio of a mixed crystal semiconductor having polarity may be used as the second conductivity type semiconductor.

When the second conductivity type semiconductor constituting the second conductivity type semiconductor layer 44 is a p-type semiconductor, for example, AlGaN doped with Mg at a concentration of 3×10¹⁹ cm⁻³ can be used as the second conductivity type semiconductor. When the second conductivity type semiconductor is a p-type semiconductor, from the viewpoint of reducing contact resistance with the LED electrode 45, the second conductivity type semiconductor layer 44 may have an AlGaN gradient composition in which an Al/(Al+Ga) ratio decreases continuously or stepwise in a direction away from the LED substrate 41. Further, from the viewpoint of suppressing electrons and holes that reduce light emission efficiency from moving, a barrier layer (not illustrated) having a large bandgap may be provided on the active layer 43 side of the second conductivity type semiconductor layer 44. Further, from the viewpoint of reducing contact resistance with the LED electrode 45, a contact layer (not illustrated) doped with a large amount of impurities may be provided on the LED electrode 45 side of the second conductivity type semiconductor layer 44.

### <LED electrode>

The LED electrode 45 is preferably formed of a material having an ohmic connection with a semiconductor layer to be in contact therewith.

Ti, Al, Ni, Au, Cr, V, Zr, Hf, Nb, Ta, Mo, W, an alloy thereof, ITO, or the like can be used as a material constituting the LED electrode 45 (for example, the LED electrode 45A in FIG. 4) in contact with an n-type semiconductor layer, and a material including aluminum and nickel is more preferable from the viewpoint of reducing contact resistance.

Ni, Au, Pt, Ag, Rh, Pd, Pt, Cu, an alloy thereof, ITO, or the like can be used as a material constituting the LED electrode 45 (for example, the LED electrode 45B in FIG. 4) in contact with the p-type semiconductor layer. When the p-type semiconductor layer is a nitride semiconductor layer, Ni, Au, an alloy thereof, or ITO, which has low contact resistance with the nitride semiconductor layer, is preferable.

So as to be connected to the LED electrode 45, metal layers of, for example, Au, Al, Cu, Ag, and W may be continuously formed, and Au with high conductivity can be desirable to be used therefor. Further, in order to improve adhesion, Ti may be further included in an interface with the LED thin film 40.

### <Transmission control unit>

In the light emitting device (LED) according to the present embodiment, a transmission control unit (not illustrated in the drawings) that controls transmission of ultraviolet light may be provided on a surface other than the first main surface 41A (a surface on which the LED thin film 40 is formed) of the LED substrate 41. The transmission control unit may be disposed at least a part of surfaces (that is, the second main surface 41B opposite to the first main surface 41A of the LED substrate 41, and a side surface 41C of the LED substrate 41) other than the first main surface 41A of the LED substrate 41. The transmission control unit transmits at least light having a central wavelength of a light emission spectrum of the active layer 43 therethrough. The transmission control unit has a function of preventing the LED substrate 41 from being degraded by suppressing the LED substrate 41 to come into contact with an external world.

The transmission control unit is not limited in particular as long as the transmission control unit is formed of a material that transmits light having a central wavelength less than 230 nm therethrough, but may be formed of a material that does not transmit unnecessary light in a wavelength band other than the central wavelength, depending on usage and the like.

From the viewpoint of efficiently transmitting light emitted from the active layer 43, the transmission control unit is preferably formed of a material having a wavelength of ± 5 nm with respect to the central wavelength of the light emitted from the active layer 43. Further, depending on purposes, it can also be preferable to transmit light having a wavelength of ± 10 nm with respect to the central wavelength. Here, "transmits light having a wavelength of x nm" means that an absorption rate of light to the wavelength of x nm is 30% or less. Further, the transmission control unit may be configured not to transmit light having a wavelength other than ± 5 nm with respect to the central wavelength, depending on usage and the like.

Further, from the viewpoint of efficiently transmitting the light emitted from the active layer 43, it is preferable to be formed of a material that transmits light having a wavelength in a half-price width band based on the central wavelength of the light emitted from the active layer 43. Further, depending on usage and the like, it may be configured not to transmit light having a wavelength other than the wavelength in the half-price width band based on the central wavelength of the light emitted from the active layer 43.

From the viewpoint of an easy optical design for transmitting light having a central wavelength of a light emission spectrum of the active layer 43, it can be preferable that the transmission control unit is a dielectric multilayer film. In one example, when the central wavelength of the light emission spectrum is 226 nm, the transmission control unit may be preferably configured with a dielectric multilayer film in which SiO₂ layers each having a thickness of 41 nm and HfO₂ layers each having a thickness of 34 nm are repeatedly stacked for seven cycles. The dielectric multilayer film can be formed by, for example, a sputtering method. Further, by stacking different materials, it is possible to suppress degradation of a material, which is one of two materials and performs a faster chemical reaction with an external component (oxygen, water, or the like) due to the chemical reaction with the external component near a surface of the second main surface 41B, and to suppress the external component to reach the second main surface 41B of the LED substrate 41.

From the viewpoint of efficiently suppressing degradation of light emission efficiency of the LED substrate 41, the transmission control unit is preferably disposed on the second main surface 41B opposite to the first main surface 41A of the LED substrate 41. Further, from the viewpoint of efficiently suppressing degradation of light emission efficiency of the LED substrate 41, the transmission control unit is preferably disposed on the side surface 41C of the LED substrate 41.

When the transmission control unit is provided, it is possible to suppress of degradation of the LED substrate 41 due to heat generated when power is applied to an LED to emit light and to suppress degradation of a surface of the LED substrate 41 due to a film (an oxide film, a hydroxide film, or the like) formed in a part of the LED substrate 41. Further, it is possible to suppress reduction of light emission efficiency of light having a desirable wavelength because the degraded portion of the surface of the LED substrate 41 reflects or absorbs light having a central wavelength of a light emission spectrum.

Meanwhile, since, in an LED which is a light emitting device of the first embodiment illustrated in FIG. 2, a surface of the LED substrate 41 is covered by the transmission control unit, the LED substrate 41 is degraded by heat generated when power is applied to emit light, and a film (an oxide film, a hydroxide film, or the like) is formed on a part of the LED substrate 41 to suppress formation of a degradation portion on a surface of the LED substrate 41. Further, the LED can output light having a desirable wavelength more efficiently, and thus, it is possible to suppress reduction of light emission efficiency due to driving.

### (1.4) Effect of present embodiment

An ultraviolet ray receiving and emitting device according to the present embodiment has following effects.
(1) An ultraviolet ray receiving and emitting device according to the present embodiment includes a light source that emits light having a wavelength of 220 nm or more and less than 230 nm, a filter that attenuates light in a wavelength band of 220 nm or more and less than 230 nm, and a light receiving portion that has sensitivity to both light in a wavelength band of 220 nm or more and less than 230 nm and light in a wavelength band of 230 nm or more and less than 400 nm, a band of light transmitting through the filter overlaps at least a part of a band in which the light receiving portion has sensitivity.
   Thereby, in a space where light in a wavelength band of a wavelength of 220 nm or more and less than 230 nm is used, when ultraviolet light that is harmful to a human body and has a wavelength band of 230 nm to 400 nm is emitted, danger can be detected by efficiently detecting ultraviolet light in a wavelength band of 230 nm to 400 nm.
(2) In the filter in the ultraviolet ray receiving and emitting device according to the present embodiment, transmissivity of light having a wavelength of 220 nm or more and less than 230 nm is one-tenth or less of transmissivity of light having a wavelength of 230 nm or more and less than 400 nm.
   Thereby, intensity of ultraviolet light having a wavelength of 220 nm or more and less than 230 nm is reduced, and thus, ultraviolet light having a wavelength of 230 nm or more and less than 400 nm can be received and detected more efficiently.
(3) In the filter of the ultraviolet ray receiving and emitting device according to the present embodiment, transmissivity increases in the order of light having a wavelength of 220 nm or more and less than 230 nm, light having a wavelength of 230 nm or more and less than 240 nm, and light having a wavelength of 240 nm or more and less than 250 nm.
   Thereby, ultraviolet light which is highly harmful to a human body easily transmits through the filter, and the light receiving portion easily detect ultraviolet light in a harmful wavelength band.
(4) The filter of the ultraviolet ray receiving and emitting device according to the present embodiment has transmissivity, which is 1% or less, of light having a wavelength of 220 nm or more and less than 230 nm.
   Thereby, ultraviolet light in a wavelength band that is harmful to a human body can be detected more efficiently by the light receiving portion.
(5) The light receiving portion of the ultraviolet ray receiving and emitting device according to the present embodiment includes a photodiode, and the filter is disposed in contact with a surface of the photodiode.
   Thereby, it is possible to reduced costs by integrating functions of the filter and the semiconductor light receiving element, and as the filter covers a surface of the semiconductor light receiving element, it is possible to suppress corrosion of the semiconductor light receiving element by air or water.
(6) In the photodiode in the ultraviolet ray receiving and emitting device according to the present embodiment, light receiving sensitivity to light having a wavelength of 220 nm or more and less than 230 nm is higher than light receiving sensitivity to light having a wavelength of 230 nm or more and less than 400 nm.
   Thereby, a signal for detecting ultraviolet ray in the wavelength band in which ozone is generated is increased to be output, and thus, a risk can be recognized more quickly.
(7) In the ultraviolet ray receiving and emitting device according to the present embodiment, a response speed of a photodiode to ultraviolet light having a wavelength of 220 nm or more and less than 400 nm is reduced less than 1 second.
   Thereby, when ultraviolet light having a wavelength of 230 nm or more and less than 400 nm, which is harmful to a human body, is emitted, it is possible to instantly notify surrounding persons of a risk.
(8) The light source of the ultraviolet ray receiving and emitting device of the present embodiment emits both light having a wavelength of 220 nm or more and less than 230 nm and light having a wavelength of 230 nm or more and less than 400 nm.
   Thereby, the light receiving portion can detect ultraviolet light having a wavelength of 230 nm or more and less than 400 nm, which is harmful to a human body, and while checking that ultraviolet light, which is emitted from a light source, having a wavelength of 230 nm or more and less than 400 nm does not exceed an allowable threshold output, the light source can be driven.
(9) In the ultraviolet ray receiving and emitting device of the present embodiment, light emission intensity of light, which is emitted from a light source, having a wavelength of 220 nm or more and less than 230 nm is 10 times or more the light emission intensity of light having a wavelength of 230 nm or more and less than 400 nm.
   Thereby, intensity of ultraviolet light having a wavelength of 220 nm or more and less than 230 nm can be increased within an allowable range of a wavelength of 230 nm or more and less than 400 nm, which is harmful to a human body.
(10) The light receiving portion of the ultraviolet ray receiving and emitting device of the present embodiment is disposed at a position where light with intensity of one-hundredth or less of the total emission from the light source is emitted.

Thereby, it is possible to efficiently emit and use emitted light of ultraviolet ray from the light source, and ultraviolet light in a wavelength band that is harmful to a human body can be detected.

### Reference Signs List

- 1: ultraviolet ray receiving device
- 2: light receiving portion
- 3: filter
- 4: light source
- 5: ultraviolet ray receiving and emitting device
- 20: mesa structure
- 21: substrate
- 21A: first main surface
- 22, 23, 24: semiconductor stacked portion
- 25, 26: electrode
- 40: LED thin film
- 41: LED substrate
- 41A: First main surface
- 41B: second main surface
- 41C: side surface
- 42: first conductivity type semiconductor layer
- 43: active layer
- 44: second conductivity type semiconductor layer
- 45, 45A, 45B: LED electrode

## Claims

1. An ultraviolet ray receiving and emitting device (5) comprising:
a light source (4) configured to emit light having a central wavelength of 220 nm or more and less than 230 nm;
a filter (3) configured to attenuate light in a wavelength band of 220 nm or more and less than 230 nm and transmit at least a part of a wavelength band of 230 nm or more and less than 320 nm; and
a light receiving portion (2) having sensitivity to at least a part of light in a wavelength band of 220 nm or more and less than 320 nm and configured to receive light transmitted through the filter (3),
wherein a band of light that transmits through the filter (3) overlaps at least a part of a band to which the light receiving portion (2) has sensitivity,
wherein transmissivity of light having a wavelength of 220 nm or more and less than 230 nm through the filter (3) is one-tenth or less of transmissivity of light having a wavelength of 230 nm or more and less than 400 nm,
wherein the light receiving portion (2) includes a photodiode, and
the filter (3) is disposed in contact with a surface of the photodiode,
wherein the photodiode has higher light receiving sensitivity for light having a wavelength of 220 nm or more and less than 230 nm than light receiving sensitivity for light having a wavelength of 230 nm or more and less than 400 nm.

2. The ultraviolet ray receiving and emitting device (5) according to claim 1,
wherein the filter (3) has transmissivity increasing in an order of light having a wavelength of 220 nm or more and less than 230 nm, light having a wavelength of 230 nm or more and less than 240 nm, and light having a wavelength of 240 nm or more and less than 250 nm.

3. The ultraviolet ray receiving and emitting device (5) according to claim 1,
wherein transmissivity of light having a wavelength of 220 nm or more and less than 230 nm through the filter (3) is 1% or less.

4. The ultraviolet ray receiving and emitting device (5) according to claim 3,
wherein a response speed of the photodiode to ultraviolet light having a wavelength of 220 nm or more and less than 400 nm is less than 1 second.

5. The ultraviolet ray receiving and emitting device (5) according to claim 1,
wherein the light source (4) emits both light having a wavelength of 220 nm or more and less than 230 nm and light having a wavelength of 230 nm or more and less than 400 nm.

6. The ultraviolet ray receiving and emitting device (5) according to claim 5,
wherein light emission intensity of the light having the wavelength of 220 nm or more and less than 230 nm from the light source (4) is 10 times or more light emission intensity of the light having the wavelength of 230 nm or more and less than 400 nm.

## Patentansprüche

1. Ultraviolettstrahlenempfangs- und -sendevorrichtung (5), umfassend:
eine Lichtquelle (4), die dazu ausgebildet ist, Licht auszusenden, das eine Zentralwellenlänge von 220 nm oder mehr und weniger als 230 nm aufweist;
einen Filter (3), der dazu ausgebildet ist, Licht in einem Wellenlängenband von 220 nm oder mehr und weniger als 230 nm zu dämpfen und mindestens einen Teil eines Wellenlängenbands von 230 nm oder mehr und weniger als 320 nm durchzulassen; und
einen Lichtempfangsabschnitt (2), der eine Empfindlichkeit gegenüber mindestens einem Teil von Licht in einem Wellenlängenband von 220 nm oder mehr und weniger als 320 nm aufweist und dazu ausgebildet ist, durch den Filter (3) durchgelassenes Licht zu empfangen,
wobei sich ein Band von Licht, das durch den Filter (3) hindurchtritt, mit mindestens einem Teil eines Bands überlappt, gegenüber dem der Lichtempfangsabschnitt (2) eine Empfindlichkeit aufweist,
wobei die Transmissivität von Licht, das eine Wellenlänge von 220 nm oder mehr und weniger als 230 nm aufweist, durch den Filter (3) ein Zehntel oder weniger der Transmissivität von Licht ist, das eine Wellenlänge von 230 nm oder mehr und weniger als 400 nm aufweist,
wobei der Lichtempfangsabschnitt (2) eine Fotodiode einschließt, und
der Filter (3) mit einer Oberfläche der Fotodiode in Kontakt stehend angeordnet ist,
wobei die Fotodiode eine höhere Lichtempfangsempfindlichkeit für Licht, das eine Wellenlänge von 220 nm oder mehr und weniger als 230 nm aufweist, als eine Lichtempfangsempfindlichkeit für Licht aufweist, das eine Wellenlänge von 230 nm oder mehr und weniger als 400 nm aufweist.

2. Ultraviolettstrahlenempfangs- und -sendevorrichtung (5) nach Anspruch 1,
wobei der Filter (3) eine Transmissivität aufweist, die zunimmt in der Reihenfolge von Licht, das eine Wellenlänge von 220 nm oder mehr und weniger als 230 nm aufweist, Licht, das eine Wellenlänge von 230 nm oder mehr und weniger als 240 nm aufweist, und Licht, das eine Wellenlänge von 240 nm oder mehr und weniger als 250 nm aufweist.

3. Ultraviolettstrahlenempfangs- und -sendevorrichtung (5) nach Anspruch 1,
wobei die Transmissivität von Licht, das eine Wellenlänge von 220 nm oder mehr und weniger als 230 nm aufweist, durch den Filter (3) 1 % oder weniger ist.

4. Ultraviolettstrahlenempfangs- und -sendevorrichtung (5) nach Anspruch 3,
wobei eine Ansprechgeschwindigkeit der Fotodiode auf Ultraviolettlicht, das eine Wellenlänge von 220 nm oder mehr und weniger als 400 nm aufweist, weniger als 1 Sekunde ist.

5. Ultraviolettstrahlenempfangs- und -sendevorrichtung (5) nach Anspruch 1,
wobei die Lichtquelle (4) sowohl Licht, das eine Wellenlänge von 220 nm oder mehr und weniger als 230 nm aufweist, als auch Licht, das eine Wellenlänge von 230 nm oder mehr und weniger als 400 nm aufweist, aussendet.

6. Ultraviolettstrahlenempfangs- und -sendevorrichtung (5) nach Anspruch 5,
wobei die Lichtemissionsintensität des Lichts, das eine Wellenlänge von 220 nm oder mehr und weniger als 230 nm aufweist, von der Lichtquelle (4) das 10-Fache oder mehr der Lichtemissionsintensität des Lichts ist, das eine Wellenlänge von 230 nm oder mehr und weniger als 400 nm aufweist.

## Revendications

1. Dispositif de réception et d'émission de rayons ultraviolets (5) comprenant :
une source lumineuse (4) configurée pour émettre une lumière présentant une longueur d'onde centrale de 220 nm ou plus et de moins de 230 nm ;
un filtre (3) configuré pour atténuer une lumière dans une bande de longueurs d'onde de 220 nm ou plus et de moins de 230 nm et transmettre au moins une partie d'une bande de longueurs d'onde de 230 nm ou plus et de moins de 320 nm ; et
une partie de réception de lumière (2) présentant une sensibilité à au moins une partie de la lumière dans une bande de longueurs d'onde de 220 nm ou plus et de moins de 320 nm et configurée pour recevoir de la lumière transmise à travers le filtre (3),
dans lequel une bande de lumière transmise à travers le filtre (3) chevauche au moins une partie d'une bande à laquelle la partie de réception de lumière (2) présente une sensibilité,
dans lequel la transmissivité de la lumière présentant une longueur d'onde de 220 nm ou plus et de moins de 230 nm à travers le filtre (3) est d'un dixième ou moins de la transmissivité de la lumière présentant une longueur d'onde de 230 nm ou plus et de moins de 400 nm,
dans lequel la partie de réception de lumière (2) comprend une photodiode, et
le filtre (3) est disposé en contact avec une surface de la photodiode,
dans lequel la photodiode présente une sensibilité de réception à la lumière plus élevée pour une lumière présentant une longueur d'onde de 220 nm ou plus et de moins de 230 nm qu'une sensibilité de réception à la lumière pour une lumière présentant une longueur d'onde de 230 nm ou plus et de moins de 400 nm.

2. Dispositif de réception et d'émission de rayons ultraviolets (5) selon la revendication 1,
dans lequel le filtre (3) présente une transmissivité qui augmente dans l'ordre suivant : lumière présentant une longueur d'onde de 220 nm ou plus et de moins de 230 nm, lumière présentant une longueur d'onde de 230 nm ou plus et de moins de 240 nm, et lumière présentant une longueur d'onde de 240 nm ou plus et de moins de 250 nm.

3. Dispositif de réception et d'émission de rayons ultraviolets (5) selon la revendication 1,
dans lequel la transmissivité de la lumière présentant une longueur d'onde de 220 nm ou plus et de moins de 230 nm à travers le filtre (3) est de 1 % ou moins.

4. Dispositif de réception et d'émission de rayons ultraviolets (5) selon la revendication 3,
dans lequel un temps de réponse de la photodiode à une lumière ultraviolette présentant une longueur d'onde de 220 nm ou plus et de moins de 400 nm est inférieur à 1 seconde.

5. Dispositif de réception et d'émission de rayons ultraviolets (5) selon la revendication 1,
dans lequel la source lumineuse (4) émet à la fois une lumière présentant une longueur d'onde de 220 nm ou plus et de moins de 230 nm et une lumière présentant une longueur d'onde de 230 nm ou plus et de moins de 400 nm.

6. Dispositif de réception et d'émission de rayons ultraviolets (5) selon la revendication 5,
dans lequel l'intensité d'émission lumineuse de la lumière présentant la longueur d'onde de 220 nm ou plus et de moins de 230 nm provenant de la source lumineuse (4) est 10 fois ou plus supérieure à l'intensité d'émission lumineuse de la lumière présentant la longueur d'onde de 230 nm ou plus et de moins de 400 nm.
